# EUROPEAN PATENT APPLICATION

(11) **EP 4 711 456 A1**
(43) Date of publication of application: **18.03.2026**
(21) Application number: 24199757.6
(22) Date of filing: 11.09.2024
(51) Int. Cl.: C12N 15/113

(54) **METHOD FOR THE MANUFACTURE OF INDIVIDUALIZED CRISPR/CAS COMPLEXES AND INDIVIDUALIZED CRISPR/CAS COMPLEXES**

(71) Applicant: GenCC GmbH & Co. KG, 69124 Heidelberg (DE)
(72) Inventor: Wilkens, Bodo, 64258 Darmstadt (DE); Macarrón Palacios, Arturo, 64625 Bensheim (DE); Korus, Patrick, 64289 Darmstadt (DE); Heshmatpour, Najmeh, 68723 Oftersheim (DE)
(74) Representative: Kröncke, Rolf

(57) **Abstract**

The present invention relates to a method for the manufacture of individualized CRISPR/Cas complexes comprising the steps a) identifying in a tumor specimen of a human cancer patient a mutation at position 38.141.150 on chromosome 3, and b) preparing for the mutation identified in a) an individualized CRISPR/Cas complex, wherein the individualized CRISPR/Cas complex comprises a guide RNA and a Cas endonuclease, wherein the CRISPR/Cas complex targets the mutation; a method, preferably an *in-vitro* method, for inducing cell death or impairment of cell proliferation in cancerous or pre-cancerous cells of B-cell-lymphocytes; a composition related thereto and individualized CRISPR/Cas complexes preferably for use in the treatment of B-cell-lymphoma or for inducing cell death or impairment of cell proliferation in cancerous or pre-cancerous B-cell-lymphocytes.

## Description

### Background

The present invention relates to a method for the manufacture of individualized CRISPR/Cas complexes comprising the steps a) identifying in a tumor specimen of a human cancer patient a mutation at position 38.141.150 on chromosome 3, and b) preparing for the mutation identified in a) an individualized CRISPR/Cas complex, wherein the individualized CRISPR/Cas complex comprises a guide RNA and a Cas endonuclease, wherein the CRISPR/Cas complex targets the mutation; a method, preferably an *in-vitro* method, for inducing cell death or impairment of cell proliferation in cancerous or pre-cancerous cells of B-cell-lymphocytes; a composition related thereto and individualized CRISPR/Cas complexes preferably for use in the treatment of B-cell-lymphoma or for inducing cell death or impairment of cell proliferation in cancerous or pre-cancerous B-cell-lymphocytes.

According to the World Health Organization (WHO), cancer represents nowadays the second leading cause of death globally. Despite improving comprehensive understanding of the pathomechanisms and the rapid advances in treatment over the last decades, cancer is with roughly 19.3 million new cancer cases and almost 10.0 million cancer-related deaths in 2020, the second leading cause of mortality and morbidity globally after cardiovascular diseases (1). Considering the rising world population and the annual cost of cancer care, cancer represents an evident health burden.

Cancer is a highly heterogeneous disease generally based on a complex process of progressive accumulation of genetic and molecular changes that result in abnormal growth of cells. During tumorigenesis, healthy cells successively acquire hallmark features including resistance to cell death, sustained proliferative signaling, evasion of growth suppressors, replicative immortality, induction of angiogenesis, and activation of invasion and metastasis (2).

Depending on their origin, most cancer types can be broadly classified in mesenchymal, epithelial, neuroectodermal, and hematopoietic. Hematological neoplasms may be further divided into lymphomas, leukemias, and myelomas, whereupon plenty further subtypes differing in cellular lineage, characteristics, response rate to therapy, and prognosis have been identified (3). Within the plethora of genetic alterations known to be associated with hematological malignancies, few aberrations in particular genomic regions have been identified to be recurrent among multiple cancer subtypes.

B cell lymphomas, a major subclass of lymphoma, represent around 5% of all newly diagnosed malignancies. This disease comprises a large variety of subtypes, including mantle cell lymphoma, Burkitt's lymphoma, diffuse large B cell lymphoma (DLBCL), follicular lymphoma, or multiple myeloma among others, depending on the stage of development of the B cell. DLBCL, the most frequent subtype of lymphoid malignancy, remains a clinical challenge, as 30% of the patients are not cured by standard-of-care immunochemotherapeutic regimens. On average, each DLBCL biopsy displays 70 lesions affecting coding domains (including mutations and copy number aberrations), with great interindividual variability. A sizable fraction of genes found mutated in >10% of patients.

Two different subgroups of DLBCL have been defined: Germinal center B-cell-like (GCB) DLBCL, lacking the expression of early post-GC differentiation markers, and Activated B-cell-like (ABC) DLBCL, which represents the most aggressive form of the disease. Approximately 30% of ABC-DLBCL patients harbour mutations leading to a hotspot L265P substitution in the hydrophobic core of the *MYD88* TIR domain (4). This adaptor molecule is critical for relaying signals required for cancer cell survival. The somatic variant (T→C) is located at position 38182641 at 3p22.2 in patients suffering from different lymphomas, e.g. DLBCL. This variant induces the amino acid change Leu 265 Pro (L265P) in *MYD88,* a mutation that triggers IRAK-mediated NF-kB signalling.

Current standard therapies against lymphomas and several other cancers comprise a variety of available treatments such as surgery, chemotherapy, radiation therapy, drug treatment therapy or immunotherapy among others. In general, remarkable advancements have been made during the last decades in the management of hematological malignancies. In spite of varying effectivity depending on the cancer subtype, large responsiveness has been observed for several lymphomas and leukemias upon combination of bone marrow transplantation and targeted chemotherapy. Notwithstanding initially high response rates, many patients treated with conventional strategies suffer disease relapse upon treatment interruption. This may be due to incomplete cancer remission, whereat remaining malignant cells proliferate after time, thus leading to relapse, resistance and poor prognosis (5). In addition, conventional therapies are often accompanied by severe side effects and physical pain derived from induction of high levels of cytotoxicity in healthy, mostly rapidly proliferating cells such as cells of the digestive tract, hair follicles, and bone marrow.

Alternative therapies to chemotherapy such as targeted immunotherapy have rapidly expanded since the development of the hybridoma technology in 1975. Immunotherapy takes advantage of specific cellular receptors that are overexpressed on the surface of cancer cells. Antibodies significantly enhance the specificity of cancer therapy through the selective targeting of tumor-associated antigens. Rituximab, targeting the B cell-related receptor CD20, or tafasitamab, directed against CD19, are prominent examples of FDA-approved monoclonal antibodies successfully used for the treatment of hematological neoplasms. Great efforts have been made to optimize the functionality of monoclonal antibodies, resulting in the development of a plethora of antibody constructs with different architectures, specificities, and valences. Further, the anti-tumor activity of antibodies has been enhanced through conjugation with potent effector molecules, resulting in antibody-drug conjugates. High response rates have been achieved, for instance, with brentuximab-vedotin (BV) for the treatment of relapsed or refractory Hodgkin lymphoma patients (Makita et al., 2020). On the downside, these so-called tumor antigen markers targeted by immunotherapy are usually not exclusively present on malignant cells, but also normal cells, although in a significantly lower density. Hence, therapies directed against cellular markers often lead to adverse side effects. These therapies have several potential disadvantages resulting from the opsonisation of large immune cell subsets expressing the same tumor-associated antigen. The resulting suppression of the immune system leads to a significantly increased risk of infection and viral reactivation. In some cases, B cell lymphomas continue to develop resulting in the mutation or even shut down of the surface expression of antigen markers. Consequently, cancer cells cannot be targeted anymore by standard immunotherapies and patients frequently relapse and become insensitive to treatment.

Additional approaches in the field of immunotherapy including implementation of immune checkpoint inhibitors have been investigated in clinical studies (6). Apart from immunotherapy, alternate drug therapies such as proteasome inhibitors are rapidly emerging. For example, Bortezomid (Velcade^{®}), carfilzomib (Kyprolis^{®}), and ixazomib (Ninlaro^{®}) have been FDA-approved for various hematological indications (7). Moreover, proteolysis-targeting chimeras (PROTACs) have arisen as a revolutionary class of drug (8). Further, the revolutionary conception of using RNA for the development of individualized cancer vaccines (9) (WO 2012/159754) and adoptive T cell therapy have opened new avenues. Axicabtagene (KTA-CD19, Yescarta^{®}) and tisagenlecleucel (Kymriah^{®}) are two chimeric antigen receptor (CAR) T cell technologies FDA-approved for the treatment of various lymphoma diseases. Such recent treatment options, however, often include an associated high risk of cytokine release syndrome (CRS) and severe cardiotoxic effects (10).

Overall, despite high initial response rates and promising results in frontline and new therapeutic options, the inherent difficulties of producing patient-tailored treatment strategies have impeded further progress. Often less than 25% of treated patients are benefited from the existing generalized therapies. Many patients experience severe cytotoxicity of healthy cells and imbalances in immune responses. Furthermore, these therapies are often challenged due to tumor heterogeneity, while malignant cells are often able to resist drug treatment, leading to poor response rates and unfavorable prognosis. There is a critical need for alternative and more personalized, i.e. individualized, strategies that target genomic tumor mutations, thereby selectively eliminating cancer cells while protecting healthy body cells.

Engineered nucleases, such as transcription activator-like effector nucleases (TALENs), zinc finger nucleases (ZFNs), and mega ribozymes, have remarkably raised the evolution of gene editing and genome engineering for precision medicine. Additionally, the clustered regularly interspaced short palindromic repeats (CRISPR)-associated protein Cas 9 (Cas9) has further intensified this progress, enabling the reliable and efficient modification, replacement, and insertion of DNA sequences. In bacteria and archaea, the CRISPR-Cas system identifies and processes foreign DNA or RNA fragments into CRISPR-derived RNA (crRNA), thus mounting an immune response against viral infection. Upon repeated invasion, the Cas endonuclease induces double-strand breaks (DSBs) contiguous to the protospacer adjacent motif (PAM), eradicating invading microorganisms. In the host cell, DSBs induced by the CRISPR-Cas system are repaired via non-homologues end joining (NHEJ) and homology directed repair (HDR). The error-prone mechanism of NHEJ often causes base insertions and deletions (indels), leading to frameshift mutations and premature stop codons and eventually gene loss-of function. By contrast, the rather precise mechanism of HDR applies a donor DNA template for DSB repair. Yet, both repair machineries can result in gene knockout, loss-of-function, or knockin (11).

Six different types and over 33 subtypes of CRISPR showing different properties and CRISPR-Cas loci have been identified to date (12). Engineering of the CRISPR-Cas system has allowed for a wide variety of genomic applications. Moreover, multiple strategies for CRISPR-based high-throughput screening have been developed for the identification of novel therapeutic targets or biomarkers (13). For instance, pooled CRISPR libraries have been established and used for genome-wide identification of tumor-driving genes as well as genes involved in survival signaling pathways or drug resistance. These defined genes can be subsequently used as drug targets to impair tumor cell growth (14). Hence, a large list of potential genes has been proposed as therapeutic targets for a plethora of cancer cell lines. Alternatively, the feasibility of employing the CRISPR-Cas technology to correct genomic alterations, (e.g. point mutations) for the treatment of hereditary diseases has been successful (15). The correction of individual genomic changes may be, however, extremely challenging for cancer therapy considering the large heterogeneity present in most tumors.

To recapitulate, these platforms aim at the determination of tumor-driving genes for the development of biological drugs, the identification of genomic alterations, or the correction of particular genetic aberrations. However, successful technology is further needed aiming at the direct induction of cell death via CRISPR-Cas-mediated genome editing in a patient- and cancer-specific manner.

In 2018, Shuber A. P. described the use of the CRISPR-Cas system to induce cancer cell death via Cas endonucleases directed against mutated sequences obtained from a cancer cell, but not present in healthy cells of a subject (16). Thus, a Cas endonuclease complex may kill cancer cells harboring genomic instability, while being inert and not damaging normal cells. Shuber describes the use of CRISPR to target any sequence of a subject associated with genomic instability (i.e., gene inversion, deletion, loss of heterozygosity, and genetic rearrangement). However, the criteria for increasing specificity and target selection have not been stated. Moreover, no experimental evidence demonstrating the compositions and methods mentioned in the disclosure are provided.

WO2018175502A2 describes a method for treating cancer with Cas endonuclease complexes. However, the disclosure is focused on different mutations and no disclosures were related to the mutation on the *MYD88* gene.

Accordingly, there is a need for products and/or methods in view of novel cancer strategies which allow the discrimination between cancerous or pre-cancerous and healthy cells that may be considered as a beneficial cancer therapy. Thus, the problem underlying the present invention is to provide products and/or methods which allow novel strategies for cancer therapy and cancer prophylaxis based on the discrimination between cancerous or pre-cancerous and healthy cells. Another aim for the same purpose underlying the present invention is to provide products and/or methods for alternative and more appropriate strategies that target genomic tumor mutations, thereby selectively eliminating cancer cells while allowing the protection of healthy body cells.

### DESCRIPTION OF THE INVENTION

This need is met by the present invention with methods, compositions and complexes according to the present invention.

In a first aspect, the problem underlying the present invention is solved by a method for the manufacture of individualized CRISPR/Cas complexes comprising the steps a) identifying in a tumor specimen of a human cancer patient a mutation at position 38.141.150 on chromosome 3; b) preparing for the mutation identified in a) an individualized CRISPR/Cas complex, wherein the individualized CRISPR/Cas complex comprises a guide RNA and a Cas endonuclease, wherein the CRISPR/Cas complex targets the mutation.

The mutation as used in the method for the manufacture of individualized CRISPR/Cas complexes according to the present invention is located on a tumor driver gene. The mutation is located on the tumor driver gene *MYD88* (myeloid differentiation primary response protein). This gene is relevant for cell survival and/or proliferation. Approximately 30% of ABC-DLBCL patients harbour mutations leading to a hotspot L265P substitution in the hydrophobic core of the *MYD88* TIR domain. This adaptor molecule is critical for relaying signals required for cancer cell survival (4). It is involved in the Toll-like receptor and IL-1 receptor signaling pathway in the innate immune response (17-19). It acts via IRAK1, IRAK2, IRF7 and TRAF6, leading to NF-kappa-B activation, cytokine secretion and the inflammatory response (17, 20, 21). Furthermore, it increases IL-8 transcription (22). It is also involved in IL-18-mediated signaling pathway. It activates IRF1 resulting in its rapid migration into the nucleus to mediate an efficient induction of IFN-beta, NOS2/INOS, and IL12A genes. The somatic variant (T>C) is located at position 38.141.150 (GrCh38) on chromosome 3 (3p22.2) in patients suffering from different lymphomas, e.g. DLBCL. This variant induces the amino acid change Leu 265 Pro (L265P) in *MYD88,* a mutation that triggers IRAK-mediated NF-kB signalling. The mutation can be found in mutated *MYD88* genes in cancer cells and not in wild-type *MYD88* genes in healthy (i.e. non-cancerous or non-pre-cancerous) genes. The sequence of the human *MYD88* gene is provided by UniProtKB - Q99836 (*MYD88*_HUMAN) as disclosed in SEQ ID NO 1. As throughout this application, any data base code used refers to the sequence available from said data base on the first priority date relating to said application.

In the human genome, the *MYD88* variant (T>C) at position 38.141.150 (GrCh38) is located on chromosome 3 (3p22.2). A variant herein means a mutation at the specific position in the respective gene in comparison to the specific position in its wild-type in a healthy (i.e. non-cancerous or non-pre-cancerous) gene. Variant T>C at position 38.141.150 on chromosome 3 creates a new protospacer adjacent motif (PAM) that can be detected by the Cas9 enzyme, among other enzymes. Concretely, this new PAM consists of the nucleotides 5'-NGG-3' in the negative DNA strand, being N any nucleotide and being guanine (G) at the second position the hotspot mutation on position 38.141.150.

According to the present invention cancerous cells are cells that divide continually, forming solid tumors or flooding the blood or lymph with abnormal cells. Cell division is a normal process used by the body for growth and repair. A parent cell divides to form two daughter cells, and these daughter cells are used to build new tissue or to replace cells that have died because of aging or damage. Healthy cells stop dividing when there is no longer a need for more daughter cells, but cancerous cells continue to produce copies. Precancerous cells are abnormal cells that could undergo changes and turn into cancer cells as time goes by. Pre-cancerous cells means there are cells that have grown abnormally, causing their size, shape or appearance to look different than normal, i.e. healthy, cells.

In contrast, according to the present invention, normal cells, also cited as healthy cells herein, are cells that are not cancerous or pre-cancerous cells. Preferably, normal/healthy cells are cells that does not include the mutations as described herein on the respective chromosome.

In a preferred embodiment, the guide RNA for the individualized CRISPR/Cas complex is equal to or is complementary to a polynucleotide with the nucleotide sequence as shown in SEQ ID NO 2 or a part thereof. It is further preferred that the mutation at position 38.141.150 is enclosed in the nucleotide sequence as shown in SEQ ID NO 2 or in the part thereof or within the new emerging PAM for the respective Cas enzyme. To be more specific, each guide RNA according to the present invention has to include or has to be complementary to the nucleotide sequence as shown in SEQ ID NO 2 or part thereof, including the nucleotide or complementary nucleotide according to the mutation at position 38.141.150 on chromosome 3 or has to include or has to be complementary to the nucleotide sequence as presented by the PAM of the respective Cas enzyme.

According to the present invention, the corresponding Cas enzyme, preferably a Cas9 enzyme, will recognize the new PAM present uniquely in precancerous or cancerous cells due to the mutation at position 38.141.150, leading to gene editing via a DSB. Consequently, gene editing may cause cellular DNA damage, preferably inducing impairment of cancer cell proliferation or cancer cell death.

In a preferred embodiment according to the present invention, the guide RNA is equal to or is complementary to a polynucleotide with the nucleotide sequence as present from position 38.141.118 to 38.141.182, preferably as present from position 38.141.152 to 38.141.171, on chromosome 3 or part therof.

In a further preferred embodiment, preferably acccording to any of the preceding embodiments, the guide RNA comprises from 64 to 16 nucleotides, preferably from 40 to 18 nucleotides, more preferably from 36 to 20 nucleotides, even more preferably from 24 to 20 nucleotides.

In a preferred embodiment, the guide RNA comprises
i) a crRNA and
ii) a tracrRNA.

Furthermore, it is preferred, that the crRNA comprises or consists of a sequence according to SEQ ID NO 3 or a variant therof or a sequence according to SEQ ID NO 4 or a variant therof. It is further preferred, that the crRNA comprises from 24 to 20 nucleotides.

Furthermore, it is preferred that the crRNA comprises or consists of a sequence according to or in each case of a variant thereof:

| **Cas enzyme** | **PAM (5'-3')** | **SEQ ID NO (CrRNA)** | **CrRNA sequence (5'-3')** |
|---|---|---|---|
| SpCas9 from *Streptococcus pyogenes* | NGG | SEQ ID NO 16 (Cr069) | GGGGATCGGTCGCTTCTGAT |
| | | SEQ ID NO 17 (Cr070) | TGGGGATCGGTCGCTTCTGA |
| | | SEQ ID NO 18 (Cr071) | ACCGATCCCCATCAAGTACA |
| | | SEQ ID NO 19 (Cr072) | ATTGCCTTGTACTTGATGGGGATCA |
| NmCas9 from *Neisseria meningitidis* | NNNNGMTT (M=A or C) | SEQ ID NO 20 (Cr073) | TGATGGGGATCGGTCGCTTCTGA |
| CjCas9 from *Campylobacter jejuni* | NNNVRYAC (V=G or C or A, R=A or G, Y=C or T) | SEQ ID NO 21 (Cr074) | CATCAGAAGCGACCGATCCCCAT |
| | | SEQ ID NO 22 (Cr075) | TGATGGGGATCGGTCGCTTCTG |
| CjCas9 from *Campylobacter jejuni* | NNNNRYAC (R=A or G, Y=C or T) | SEQ ID NO 23 (Cr076) | CATCAGAAGCGACCGATCCCCA |
| | | SEQ ID NO 24 (Cr077) | TTNCAGGTGCCCATCAGAAGCGACCG |
| FnCpf1 from *Francisella* | TTN | SEQ ID NO 25 (Cr078) | TTNATGGGGATCGGTCGCTTCTGATG |
| | | SEQ ID NO 26 (Cr079) | TTNTACTTGATGGGGATCGGTCGCTT |
| | | SEQ ID NO 27 (Cr080) | TTNCCTTGTACTTGATGGGGATCGGT |
| | | SEQ ID NO 28 (Cr081) | GCAGGTGCCCATCAGAAGCG |
| SaCas9 from *Staphylococcus aureus* | NNNRRT (R=A or G) | SEQ ID NO 29 (Cr082) | CTTGATGGGGATCGGTCGCT |
| | | SEQ ID NO 30 (Cr083) | TCAGAAGCGACCGATCCCCA |
| | | SEQ ID NO 31 (Cr084) | TTGCCTTGTACTTGATGGGG |
| | | SEQ ID NO 32 (Cr085) | CCGATCCCCATCAAGTACAA |
| | | SEQ ID NO 33 (Cr086) | AGGTGCCCATCAGAAGCGAC |
| VQR SpCas9 from Streptococcus pyogenes: | NGA | SEQ ID NO 34 (Cr087) | TGATGGGGATCGGTCGCTTC |
| | | SEQ ID NO 35 (Cr088) | GAAGCGACCGATCCCCATCA |
| xCas9 3.7 (TLIKDIV SpCas9) from *Streptococcus pyogenes* | NGT | SEQ ID NO 36 (Cr089) | CCTTGTACTTGATGGGGATC |
| | | SEQ ID NO 37 (Cr090) | CAGGTGCCCATCAGAAGCGACCG |
| BhCas12b from *Bacillus hisashii* | TTN | SEQ ID NO 38 (Cr091) | ATGGGGATCGGTCGCTTCTGATG |
| | | SEQ ID NO 39 (Cr092) | TACTTGATGGGGATCGGTCGCTT |
| | | SEQ ID NO 40 (Cr093) | CCTTGTACTTGATGGGGATCGGT |
| | | SEQ ID NO 41 (Cr094) | CCTTGTACTTGATGGGGATCGGT |
| BhCas12b v4 from *Bacillus hisashii* | ATTN | SEQ ID NO 42 (Cr095) | CCTTGTACTTGATGGGGATCGGT |
| BhCas12b v4 from *Bacillus hisashii* | DTTN | SEQ ID NO 43 (Cr096) | AGGTGCCCATCAGAAGCGAC |
| Nme2Cas9 from *Neisseria meningitidis* | NNNNCC | SEQ ID NO 44 (Cr097) | GGTGCCCATCAGAAGCGACC |
| | | SEQ ID NO 45 (Cr098) | GGGATCGGTCGCTTCTGATG |
| | | SEQ ID NO 46 (Cr099) | GTGCCCATCAGAAGCGACCG |
| | | SEQ ID NO 47 (Cr100) | TTGCCTTGTACTTGATGGGGATCG |
| RR AsCpf1 from *Acidaminococcus* | TYCV | SEQ ID NO 48 (Cr101) | ATCGGTCGCTTCTGATGGGC |
| CcCas9 from *Clostridium cellulolyticum* | NNNNGNA | SEQ ID NO 49 (Cr102) | ATGGGGATCGGTCGCTTCTG |
| | | SEQ ID NO 50 (Cr103) | TCAGAAGCGACCGATCCCCA |
| | | SEQ ID NO 51 (Cr104) | CGACCGATCCCCATCAAGTA |
| | | SEQ ID NO 52 (Cr105) | CCCATCAGAAGCGACCGATCCCC |
| ShCas12k from *Scytonema hofmanni* | GTN | SEQ ID NO 53 (Cr106) | CTTGATGGGGATCGGTCGCTTCT |
| | | SEQ ID NO 54 (Cr107) | TCGGTCGCTTCTGATGGGCA |
| ThermoCas9 from *Geobacillus thermodenitrificans T1* | NNNNCNR | SEQ ID NO 55 (Cr108) | GGATCGGTCGCTTCTGATGG |
| | | SEQ ID NO 56 (Cr109) | GTGCCCATCAGAAGCGACCG |
| | | SEQ ID NO 57 (Cr110) | CCATCAGAAGCGACCGATCC |
| | | SEQ ID NO 58 (Cr111) | GTACTTGATGGGGATCGGTC |
| | | SEQ ID NO 59 (Cr112) | GAAGCGACCGATCCCCATCA |
| | | SEQ ID NO 60 (Cr113) | GACCGATCCCCATCAAGTAC |
| | | SEQ ID NO 61 (Cr114) | CATTGCCTTGTACTTGATGG |
| | | SEQ ID NO 62 (Cr115) | ATGGGGATCGGTCGCTTCTG |
| Cas12f1 from *Acidibacillus sulfuroxidans* | NTTR | SEQ ID NO 63 (Cr116) | TACTTGATGGGGATCGGTCG |
| | | SEQ ID NO 64 (Cr117) | GGTGCCCATCAGAAGCGACC |
| SpCas9 Variant (TAT.P5-1) from *Streptococcus pyogenes* (R=G or A, H=A or C orT) | NRTH | SEQ ID NO 65 (Cr118) | CATCAGAAGCGACCGATCCC |
| | | SEQ ID NO 66 (Cr119) | GAAGCGACCGATCCCCATCA |
| | | SEQ ID NO 67 (Cr120) | CCTTGTACTTGATGGGGATC |
| | | SEQ ID NO 68 (Cr121) | TGGGGATCGGTCGCTTCTGA |
| SpCas9 from *Staphylococcus Auricularis* | NNGG | SEQ ID NO 69 (Cr122) | ATGGGGATCGGTCGCTTCTG |
| | | SEQ ID NO 70 (Cr123) | GACCGATCCCCATCAAGTAC |
| | | SEQ ID NO 71 (Cr124) | CAGAAGCGACCGATCCCCATCA |
| Frcas9 from *Faecalibaculum rodentium* (R=A or G) | NRTA | SEQ ID NO 72 (Cr125) | TGGGGATCGGTCGCTTCTGA |
| LtCas12a from Cas12a family | TTNA | SEQ ID NO 73 (Cr126) | GGTCGCTTCTGATGGGCACC |
| | | SEQ ID NO 74 (Cr127) | AGGTGCCCATCAGAAGCGAC |
| | | SEQ ID NO 75 (Cr128) | GGTGCCCATCAGAAGCGACC |
| | | SEQ ID NO 76 (Cr129) | GATCGGTCGCTTCTGATGGG |
| | | SEQ ID NO 77 (Cr130) | GGGATCGGTCGCTTCTGATG |
| SpCas9 | NRNH | SEQ ID NO 78 (Cr131) | GGGGATCGGTCGCTTCTGAT |
| | | SEQ ID NO 79 (Cr132) | CATCAGAAGCGACCGATCCC |
| | | SEQ ID NO 80 (Cr133) | TGATGGGGATCGGTCGCTTC |
| | | SEQ ID NO 81 (Cr134) | AGAAGCGACCGATCCCCATC |
| | | SEQ ID NO 82 (Cr135) | GAAGCGACCGATCCCCATCA |
| | | SEQ ID NO 83 (Cr136) | AGCGACCGATCCCCATCAAG |
| | | SEQ ID NO 84 (Cr137) | TGTACTTGATGGGGATCGGT |
| | | SEQ ID NO 85 (Cr138) | CCTTGTACTTGATGGGGATC |
| | | SEQ ID NO 86 (Cr139) | ACCGATCCCCATCAAGTACA |
| | | SEQ ID NO 87 (Cr140) | GCCTTGTACTTGATGGGGAT |
| | | SEQ ID NO 88 (Cr141) | CCGATCCCCATCAAGTACAA |
| | | SEQ ID NO 89 (Cr142) | TGGGGATCGGTCGCTTCTGA |
| VRQR SpCas9 from *Streptococcus pyogenes* | NGNG | SEQ ID NO 90 (Cr143) | TGCAGGTGCCCATCAGAAGC |
| SpCas9 Variant from *Streptococcus pyogenes* | NRCH | SEQ ID NO 91 (Cr144) | GGTCGCTTCTGATGGGCACC |
| | | SEQ ID NO 92 (Cr145) | GATCGGTCGCTTCTGATGGG |
| | | SEQ ID NO 93 (Cr146) | GGGATCGGTCGCTTCTGATG |
| | | SEQ ID NO 94 (Cr147) | AGCGACCGATCCCCATCAAG |
| | | SEQ ID NO 95 (Cr148) | TGTACTTGATGGGGATCGGT |
| | | SEQ ID NO 96 (Cr149) | CCGATCCCCATCAAGTACAA |

In a preferred embodiment according to the present invention, the guide RNA for the individualized CRISPR/Cas complex specifically binds to a polynucleotide with the nucleotide sequence as shown in SEQ ID NO 2 or the complementary sequence of SEQ ID NO 2 or a part of those sequences. It is further preferred that the guide RNA for the individualized CRISPR/Cas complex specifically binds to a polynucleotide with the nucleotide sequence as shown in SEQ ID NO 3 or 4 or the complementary sequence of SEQ ID NO 3 or 4.

A tracrRNA according to the present invention can be each tracrRNA that is suitable. A preferred tracrRNA has the sequence GUUUUAGAGC UAUGCU according to SEQ ID NO 5. This tracrRNA sequence is part of SEQ ID NOs 6 to 9 as disclosed below.

In a preferred embodiment according to the present invention, the guide RNA comprises or consists of a sequence according to SEQ ID NO 6 or a variant therof or a sequence according to SEQ ID NO 7 or a variant therof.

In a preferred embodiment, the term "variant", as used herein, may refer to at least one fragment of the full length sequence referred to, more specifically one or more amino acid or nucleic acid sequence which is, relative to the full-length sequence, truncated at one or both termini by one or more amino acids. Such a fragment comprises at least 6, 7, 8, 10, 12, 15, 20, 25, 50 or 60 successive amino acids of the original sequence or a variant thereof. The total length of the variant may be at least 6, 7, 8, 9, 10, 11, 12, 20, 25, 30, 40, 50 or 60 or more amino acids. In preferred alternative embodiments, the variant has a length of not more than 30, not more than 28, not more than 26, not more than 24 amino acids.

In other preferred embodiments, the variant has at least 80%, at least 90%, at least 95%, at least 98% sequence identity to the sequence referred to. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (23) is used using default settings.

In a preferred embodiment, the variant may, in addition, comprise chemical modifications, for example isotopic labels or covalent modifications such as glycosylation, phosphorylation, acetylation, decarboxylation, citrullination, methylation, hydroxylation and the like. The person skilled in the art is familiar with methods to modify. Any modification is designed such that it does not abolish the biological activity of the variant.

Preferably, the variant has biological activity. In a preferred embodiment, such biological activity is the ability to bind specifically to a polynucleotide with the nucleotide sequence as shown in SEQ ID NO 2 or the complementary sequence of SEQ ID NO 2 or a part of those sequences, more preferably the ability to bind specifically to a polynucleotide with the nucleotide sequence as shown in SEQ ID NO 3 or 4 or the complementary sequence of SEQ ID NO 3 or 4.

The term "individualized CRISPR/Cas complex" used herein has the meaning that this CRISPR/Cas complex is designed based on individual information, i.e. on one mutation at a position in the sequence on a chromosome in cancerous or pre-cancerous cells compared to the wild type sequence on the chromosome in healthy, i.e. non-cancerous, cells, obtained from a human cancer patient or a patient supposed to have or to develop cancer in the near future. It has to be noted that not every human cancer patient shows the mutation at position 38.141.150 on chromosome 3. Only those having this mutation will benefit from an administration of the individualized CRISPR/Cas complexes of the present invention, preferably manufactured according to a method for the manufacture of individualized CRISPR/Cas complexes of the present invention and further methods, complexes and compositions according to the present invention as disclosed herein.

According to the present invention, the term "the CRISPR/Cas complex targets the mutation" used herein has the meaning that the CRISPR/Cas complex binds to the mutation at position 38.141.150 on chromosome 3 or recognizes the PAM of a respective Cas enzyme, wherein the PAM is newly created by the mutation. This is specifically the case if Cas9 is used. The PAM of Cas9 is NGG. This PAM does not exist in the wild-type sequence on chromosome 3 in the area of the potential mutation at position 38.141.150 in healthy, i.e. non-cancerous, cells. However, the mutation at position 38.141.150 on chromosome 3 is located within the PAM recognized by Cas9, i.e. the nucleotides NGG. For other Cas enzymes, its corresponding PAM may be located +/- 30 positions upstream or downstream from the mutation at position 398.141.150. A CRISPR/Cas complex according to the present invention preferably includes a crRNA sequence comprising a sequence that is equal to or is complementary to the new DNA sequence.

Each guide RNA prepared according to the present invention is directed against a nucleic acid sequence that is specific for the mutation occuring in cancerous or pre-cancerous cells compared to the nucleic acid sequence at the same position occuring in healthy, i.e. non-cancerous or non-pre-cancerous, cells.

According to the present invention, an individualized CRISPR/Cas complex preferably comprises the guide RNA and the Cas endonuclease in a ratio of 3:1 to 1:1.

According to the present invention, the Cas endonuclease is preferably a type I, type II or type III endonuclease, more preferably a type I endonuclease, even more preferably a CRISPR/Cas endonuclease, most preferably a CRISPR/Cas9 endonuclease (Cas9). Any Cas endonuclease may be used in the disclosed methods, complexes and compositions. For example, the Cas endonuclease may be a Cas9 and cut DNA. A complex including a Cas9 endonuclease may be referred to as a CRISPR/Cas9 complex. In another example, the Cas endonuclease may be a Cas12a and cut RNA. A complex including the Cas12a endonuclease may be referred to as a CRISPR/Cas12a complex.

In a further preferred embodiment of the present invention, the tumor specimen is preferably from glioma cells, astrocytoma cells, neuroblastoma cells, meningioma cells, glioblastoma cells, urothelial carcinoma cells, bladder papilloma cells, rhabdomyosarcoma cells, osteosarcoma cells, Ewing's sarcoma cells, chondrosarcoma cells, mammary carcinoma cells, breast adenocarcinoma cells, squamous carcinoma cells, cervical adenocarcinoma cells, choriocarcinoma cells, leiomyosarcoma cells, colorectal adenocarcinoma cells, colon carcinoma cells, hepatoblastoma cells, hepatic adenocarcinoma cells, gall bladder adenocarcinoma cells, epithelial appendiceal cancer cells, neuroendocrine appendiceal cancer cells, goblet cell carcinomas, cholangiocarcinoma cells, gastrointestinal carcinoid tumor cells, pancreas adenocarcinoma cells, neuroendocrine carcinoma cells, renal adenocarcinoma cells, renal carcinoma cells, pharynx carcinoma cells, neck squamous carcinoma cells, thyroid carcinoma cells, nasal squamous carcinoma cells, oesophagus squamous cells, submaxillary gland adenoma cells, cone precursors cells, neuroendocrine cells, prostate luminal cells, prostatic adenocarcinoma cells, pancreas adenocarcinoma cells, cervix carcinoma cells, lung carcinoma cells, lung squamous cell carcinoma (SCC) cells, lung epidermoid carcinoma cells, hepatoblastoma (hepatocellular carcinoma) cells, hepatic adenocarcinoma cells, Alexander hepatoma cells, ovary adenocarcinoma cells, endometrium adenocarcinoma cells, basal carcinoma cells, skin squamous carcinoma cells or B-cell-lymphocytes, most preferably from B-cell-lymphocytes.

A tumor specimen according to the present invention may have been obtained in any clinically acceptable manner.

According to the present invention, "specimen" and "sample" are used interchangeable having the same meaning.

In a second aspect, the problem underlying the present invention is solved by a method, preferably by an *in-vitro* method, for inducing cell death in cancerous or pre-cancerous cells of B-cell-lymphocytes, comprising the steps:
a) isolation of B-cell-lymphocytes from one or more specimen obtained from a human cancer patient or a human subject supposed to have or to develop cancer, whereby non-cancerous B-cell-lymphocytes and cancerous or pre-cancerous B-cell-lymphocytes are obtained;
b) cultivation, preferably *in-vitro* cultivation, of the B-cell-lymphocytes from the one or more specimen obtained in a);
c) identifying in the cancerous or pre-cancerous B-cell-lymphocytes a cancer specific mutation at position 38141150 on chromosome 3;
d) preparing an individualized CRISPR/Cas complex for the cancer specific mutation identified in c) according to step b) of the method for the manufacture of individualized CRISPR/Cas complexes according to the present invention and preferably as described in at least one of the embodiments as disclosed herein,
e) the prepared individualized CRISPR/Cas complex to the cultivated B-cell-lymphocytes obtained in step b), and
f) optionally, determining if cell death or impairment of cell proliferation is induced in the cancerous or pre-cancerous B-cell-lymphocytes of said cultivated B-cell-lymphocytes and/or preferably determining if cell death or impairment of cell proliferation is not induced in the non-cancerous B-cell-lymphocytes of said cultivated B-cell-lymphocytes.

The one or more specimen from a human cancer patient or a human subject supposed to have or to develop cancer have been obtained before the steps of the method, preferably the *in-vitro* method, for inducing cell death in cancerous or pre-cancerous cells of B-cell-lymphocytes according to the present invention are performed. A specimen may have been obtained in any clinically acceptable manner.

According to the present invention, cell death is preferably induced by gene knockout as a consequence of double strand breaks. Furthermore, according to the present invention, the induction of cell death is preferably reached through apoptosis. Alternatively, the gene knockout may lead to impairment of cell proliferation.

In a preferred embodiment of this aspect, step f) further includes determining if cell death is not induced in the non-cancerous B-cell-lymphocytes of said cultivated B-cell-lymphocytes. It is further preferred that cell death is induced in the cancerous or pre-cancerous B-cell-lymphocytes of said cultivated B-cell-lymphocytes only.

In a third aspect, the problem underlying the present invention is solved by an individualized CRISPR/Cas complex comprising a guide RNA according to the present invention and a Cas endonuclease, preferably a Cas9 endonuclease, wherein the guide RNA is equal to or is complementary to a polynucleotide with the nucleotide sequence as shown in SEQ ID NO 2 or a part thereof.

In a preferred embodiment, the individualized CRISPR/Cas complex according to the present invention comprises or consists of a
i) a crRNA according to SEQ ID NO 3 or a variant therof or a crRNA according to SEQ ID NO 4 or a variant therof and
il) a tracrRNA.

In a fourth aspect, the problem underlying the present invention is solved by a composition, preferably a pharmaceutical composition, comprising one or more individualized CRISPR/Cas complexes manufactured according to the method for the manufacture of individualized CRISPR/Cas complexes according to the present invention, preferably according to at least one of the embodiments as described herein.

In a fifth aspect, the problem underlying the present invention is solved by an individualized CRISPR/Cas complex manufactured according to the method for the manufacture of individualized CRISPR/Cas complexes according to the present invention, an individualized CRISPR/Cas complex according to the present invention or a composition, preferably a pharmaceutical composition, comprising one or more of such individualized CRISPR/Cas complexes, for use in the treatment of B-cell-lymphoma, preferably for use in the treatment of diffuse large B-cell-lymphoma, more preferably for use in the treatment of Germinal center B-cell-like diffuse large B-cell-lymphoma or Activated B-cell-like diffuse large B-cell-lymphoma.

The inventors of the present invention have developed individualized CRISPR/Cas complexes of the present invention as described herein which have surprisingly shown that they can induce cell death in cancerous or pre-cancerous B-cell-lymphocytes only but not in healthy, i.e. non-cancerous or non-pre-cancerous, B-cell-lymphocytes. Accordingly, these individualized CRISPR/Cas complexes according to the invention are or at least seem to be suitable for the use in the treatment of B-cell-lymphoma.

Preferably, an individualized CRISPR/Cas complex or a composition according to the present invention may be administered using any amount and any route of administration effective for treating cancer. In a further preferred embodiment, such compositions complex or composition may be administered in an amount effective for treating a cancer. Thus, the expression "amount effective for treating a cancer", as used herein, refers to a sufficient amount of composition to beneficially prevent or ameliorate the symptoms of the cancer.

The exact dosage for a individualized CRISPR/Cas complex or composition according to the present invention to be used for cancer therapy may be chosen by an individual physician in view of the subject to be treated and certain other factors. Dosage and administration are adjusted to provide sufficient levels of the composition or to maintain the desired effect. Additional factors which may be taken into account include the severity of the disease state, age, weight and gender of the subject; diet, time and frequency of administration; route of administration; drug combinations; reaction sensitivities; and tolerance/response to therapy. Long acting pharmaceutical compositions might be administered, for example, hourly, twice hourly, every 3 to four hours, daily, twice daily, every 3 to 4 days, every week, or once every two weeks depending an half-life and clearance rate of the particular composition. The disclosed compositions may preferably be formulated in dosage unit form for ease of administration and uniformity of dosage. The expression "dosage unit form" as used herein refers to a physically discrete unit of an inventive composition appropriate for the subject to be treated. It will be understood, however, that the total daily usage of the disclosed compositions will be decided by the attending physician within the scope of sound medical judgment. For any active agent, the therapeutically effective dose may be estimated initially either in cell culture assays or in animal models, usually mice, but also potentially from rats, rabbits, dogs, or pigs.

In a preferred embodiment, an individualized CRISPR/Cas complex or composition according to the present invention is or is part of a pharmaceutical composition. As formulated with an appropriate pharmaceutically acceptable carrier in a desired dosage, such a pharmaceutical composition provided herein may be administered to humans and other mammals topically such as ocularly, nasally, bucally, orally, rectally, parenterally, intracisternally, intravaginally, or intraperitoneally. As used herein, the term "pharmaceutically acceptable carrier" includes any and all solvents, diluents, or other liquid vehicles, dispersion or suspension aids, surface active agents, isotonic agents, thickening or emulsifying agents, preservatives, solid binders, lubricants and the like, as suited to the particular dosage form desired. Remington's Pharmaceutical Sciences Ed. By Gennaro, Mack Publishing, Easton, PA, 1995, provides various carriers used in formulating pharmaceutical compositions and techniques for the preparation thereof. Some examples of materials which can serve as pharmaceutically acceptable carriers include, but are not limited to, sugars such as glucose and sucrose; excipients such as cocoa butter and suppository waxes; oils such as peanut oil, cottonseed oil, safflower oil, sesame oil, olive oil, corn oil, and soybean oil; glycols such a propylene glycol; esters such as ethyl oleate and ethyl laurate; agar; buffering agents such as magnesium hydroxide and aluminum hydroxide; alginic acid; pyrogen-free water; isotonic sahne; Ringer's solution; ethyl alcohol; and phosphate buffer solutions, as well as other non-toxic compatible lubricants such as sodium lauryl sulfate and magnesium stearate, as well as coloring agents, releasing agents, coating agents, preservatives and antioxidants can also be present in the composition, according to the judgment of the formulator. Liquid dosage forms for ocular, oral, or other systemic administration include, but are not limited to, pharmaceutically acceptable emulsions, microemulsions, solutions, suspensions, syrups and elixirs. In addition to the active agent(s), the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the ocular, oral, or other systemically-delivered compositions can also include adjuvants such as wetting agents, and emulsifying and suspending agents. Dosage forms for topical or transdermal administration of an inventive pharmaceutical composition include ointments, pastes, creams, lotions, gels, powders, solutions, sprays, inhalants, or patches. The active agent is admixed under sterile conditions with a pharmaceutically acceptable carrier and any needed preservatives or buffers as may be required. For example, ocular or cutaneous routes of administration are achieved with aqueous drops, a mist, an emulsion, or a cream. Administration may be therapeutic or it may be prophylactic. The disclosure includes ophthalmological devices, surgical devices, audiological devices or products which contain disclosed compositions (e.g., gauze bandages or strips), and methods of making or using such devices or products. These devices may be coated with, impregnated with, bonded to or otherwise treated with a composition as described herein. Transdermal patches have the added advantage of providing controlled delivery of the active ingredients to the body. Such dosage forms may be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel. Compositions for rectal or vaginal administration are preferably suppositories which can be prepared by mixing the active agent(s) of this invention with suitable non-irritating excipients or carriers such as cocoa butter, polyethylene glycol or a suppository wax which are solid at ambient temperature but liquid at body temperature and therefore melt in the rectum or vaginal cavity and release the active agent(s). Solid dosage forms for oral administration include capsules, tablets, pills, powders, and granules. In such solid dosage forms, the active agent is mixed with at least one inert, pharmaceutically acceptable excipient or carrier such as sodium citrate or dicalcium phosphate and/or a) fillers or extenders such as starches, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. Solid compositions of a similar type may also be employed as fillers in soft and hard-filled gelatin capsules using such excipients as milk sugar as well as high molecular weight polyethylene glycols and the like. The solid dosage forms of tablets, dragees, capsules, pills, and granules can be prepared with coatings and shells such as enteric coatings, release controlling coatings and other coatings suitable for pharmaceutical formulation. In such solid dosage forms the active agent(s) may be admixed with at least one inert diluent such as sucrose or starch. Such dosage forms may also comprise, as is normal practice, additional substances other than inert diluents, e.g., tableting lubricants and other tableting aids such as magnesium stearate and microcrystalline cellulose. In the case of capsules, tablets and pills, the dosage forms may also comprise buffering agents. They may optionally contain pacifying agents and can also be of a composition that they release the active agent(s) only, or preferentially, in a certain part of the intestinal tract, optionally, in a delayed manner. Examples of embedding compositions which can be used include polymeric substances and waxes.

In a sixth aspect, the problem underlying the present invention is solved by an individualized CRISPR/Cas complex manufactured according to the method for the manufacture of individualized CRISPR/Cas complexes of the present invention, an individualized CRISPR/Cas complex according to the present invention or a composition, preferably a pharmaceutical composition, comprising one or more of such individualized CRISPR/Cas complexes, for inducing cell death in cancerous or pre-cancerous B-cell-lymphocytes. According to the present invention, cell death is not induced in healthy, i.e. non-cancerous or non-pre-cancerous, B-cell-lymphocytes by the use of such one, two or more individualized CRISPR/Cas complexes or compositions comprising one, two or more of such individualized CRISPR/Cas complexes.

In a seventh aspect, the problem underlying the present invention is solved by an individualized CRISPR/Cas complex for use in the treatment of cancer, preferably for use in inducing cell death in cancerous or pre-cancerous cells of B-cell-lymphocytes, wherein the individualized CRISPR/Cas complex is manufactured according to a method, preferably according to an *in-vitro* method, comprising the steps:
a) isolation of B-cell-lymphocytes from one or more specimen obtained from a human cancer patient or a human subject supposed to have or to develop cancer, whereby non-cancerous B-cell-lymphocytes and cancerous or pre-cancerous B-cell-lymphocytes are obtained;
b) cultivation, preferably *in-vitro* cultivation, of the B-cell-lymphocytes from the one or more specimen obtained in a);
c) identifying in the cancerous or pre-cancerous B-cell-lymphocytes a cancer specific mutation at position 38.141.150 on chromosome 3,
d) preparing an individualized CRISPR/Cas complex for the cancer specific mutation identified in c) according to step b) of the method for the manufacture of individualized CRISPR/Cas complexes according to the present invention.

In certain embodiments of the present invention, sequencing may be performed by the Sanger sequencing technique. Classical Sanger sequencing involves a single-stranded DNA template, a DNA primer, a DNA polymerase, radioactively or fluorescently labeled nucleotides, and modified nucleotides that terminate DNA strand elongation. If the label is not attached to the dideoxynucleotide terminator (e.g., labeled primer), or is a monochromatic label (e.g., radioisotope), then the DNA sample is divided into four separate sequencing reactions, containing four standard deoxynucleotides (dATP, dGTP, dCTP and dTTP) and the DNA polymerase. To each reaction is added only one of the four dideoxynucleotides (ddATP, ddGTP, ddCTP, or ddTTP). These dideoxynucleotides are the chain-terminating nucleotides, lacking a 3'-OH group required for the formation of a phosphodiester bond between two nucleotides during DNA strand elongation. If each of the dideoxynucleotides carries a different label, however, (e.g., 4 different fluorescent dyes), then all the sequencing reactions can be carried out together without the need for separate reactions. Incorporation of a dideoxynucleotide into the nascent (i.e., elongating) DNA strand terminates DNA strand extension, resulting in a nested set of DNA fragments of varying length. Newly synthesized and labeled DNA fragments are denatured, and separated by size using gel electrophoresis on a denaturing polyacrylamide-urea gel capable of resolving single-base differences in chain length. If each of the four DNA synthesis reactions was labeled with the same, monochromatic label (e.g., radioisotope), then they are separated in one of four individual, adjacent lanes in the gel, in which each lane in the gel is designated according to the dideoxynucleotide used in the respective reaction, i.e., gel lanes A, T, G, C. If four different labels were utilized, then the reactions can be combined in a single lane on the gel. DNA bands are then visualized by autoradiography or fluorescence, and the DNA sequence can be directly read from the X-ray film or gel image. The terminal nucleotide base is identified according to the dideoxynucleotide that was added in the reaction resulting in that band or its corresponding direct label. The relative positions of the different bands in the gel are then used to read (from shortest to longest) the DNA sequence as indicated. The Sanger sequencing process can be automated using a DNA sequencer, such as those commercially available from PerkinElmer, Beckman Coulter, Life Technologies, and others.

In other embodiments, sequencing may be accomplished by a single-molecule sequencing by synthesis technique. Single molecule sequencing is shown for example in Lapidus et al. (U.S. patent number 7,169,560), Quake et al. (U.S. patent number 6,818,395), Harris (U.S. patent number 7,282,337), Quake et al. (U.S. patent application number 2002/0164629), and Braslavsky, et al., PNAS (USA), 100: 3960-3964 (2003). Briefly, a single-stranded nucleic acid (e.g., DNA or cDNA) is hybridized to oligonucleotides attached to a surface of a flow cell. The oligonucleotides may be covalently attached to the surface or various attachments other than covalent linking may be employed. Moreover, the attachment may be indirect, e.g., via a polymerase directly or indirectly attached to the surface. The surface may be planar or otherwise, and/or may be porous or non-porous, or any other type of surface suitable for attachment. The nucleic acid is then sequenced by imaging the polymerase-mediated addition of fluorescently-labeled nucleotides incorporated into the growing strand surface oligonucleotide, at single molecule resolution. Other single molecule sequencing techniques involve detection of pyrophosphate as it is cleaved from incorporation of a single nucleotide into a nascent strand of DNA, as is shown in Rothberg et al. (U.S. patent numbers 7,335,762, 7,264,929, 7,244,559, and 7,211,390) and Leamon et al. (U.S. patent number 7,323,305), each of which is incorporated by reference.

In other embodiments, targeted resequencing can be used. Resequencing is shown for example in Harris (U.S. patent application numbers 2008/0233575, 2009/0075252, and 2009/0197257). Briefly, a specific segment of the target is selected (for example by PCR, microarray, or MIPS) prior to sequencing. A primer designed to hybridize to this particular segment, is introduced and a primer/template duplex is formed. The primer/template duplex is exposed to a polymerase, and at least one detectably labeled nucleotide under conditions sufficient for template dependent nucleotide addition to the primer. The incorporation of the labeled nucleotide is determined, as well the identity of the nucleotide that is complementary to a nucleotide an the template at a position that is opposite the incorporated nucleotide. After the polymerization reaction, the primer may be removed from the duplex. The primer may be removed by any suitable means, for example by raising the temperature of the surface or substrate such that the duplex is melted, or by changing the Buffer conditions to destabilize the duplex, or combination thereof. Methods for melting template/primer duplexes are described, for example, in chapter 10 of Molecular Cloning, a Laboratory Manual, 3.sup.rd Edition, J. Sambrook, and D. W. Russell, Cold Spring Harbor Press (2001). After removing the primer, the template may be exposed to a second primer capable of hybridizing to the template. In one embodiment, the second primer is capable of hybridizing to the same region of the template as the first primer (also referred to herein as a first region), to form a template/primer duplex. The polymerization reaction is then repeated, thereby resequencing at least a portion of the template. If the nucleic acid from the sample is degraded or only a minimal amount of nucleic acid can be obtained from the sample, PCR can be performed on the nucleic acid in order to obtain a sufficient amount of nucleic acid for sequencing (See e.g., Mullis et al. U.S. patent number 4,683,195).

In certain embodiments of the present invention, next-generation sequencing (NGS) may be performed. This method requires DNA enrichment and library preparation. Subsequently, NGS technology involves clonal amplification and sequencing by synthesis (SBS) chemistry to simultaneously read through independent DNA templates for the identification of DNA bases and their incorporation into a nucleic acid chain. The order of the DNA sequence is generally ascertained via detection of fluorescent signal emitted by each nucleic acid base upon addition to the growing strand. Thus, short reads of often less than 500 bp with extensive depth of coverage are generated, allowing for fast sequencing in a high-throughput manner. This technology may be used for whole genome sequencing, whole exome sequencing or deep sequencing of specific target regions of healthy as well as malignant samples, among others. Post-sequencing analysis enables identification of genetic alterations such as single nucleotide polymorphisms (SNPs), copy number variations, translocations, insertions and deletions events, or for ascertainment of tumor subclones and subpopulations. NGS can be carried out by commercial suppliers such as Illumina by using different sequencers, depending on method and application.

The present invention is further illustrated by the following examples, sequences and figures from which further features, embodiments, aspects and advantages of the present invention may be taken. All methods and materials similar or equivalent to those described herein can be used in the practice or testing of the present invention, with suitable methods and materials being described herein. All publications, patent applications, patens, and other references mentioned herein are incorporated by reference in their entirety. Further, the materials, methods, and examples are illustrative only and are not intended to be limiting unless otherwise specified.
**SEQ ID NO 1 (UniProtKB Q99836 (MYD88_HUMAN))** 
MAAGGPGAGSAAPVSSTSSLPLAALNMRVRRRLSLFLNVRTQVAADWTALAEEMDFEY
LEIRQLETQADPTGRLLDAWQGRPGASVGRLLELLTKLGRDDVLLELGPSIEEDCQKYILK
QQQEEAEKPLQVAAVDSSVPRTAELAGITTLDDPLGHMPERFDAFICYCPSDIQFVQEMI
 
RQLEQTNYRLKLCVSDRDVLPGTCVWSIASELIEKRCRRMVVVVSDDYLQSKECDFQTK
FALSLSPGAHQKRLIPIKYKAMKKEFPSILRFITVCDYTNPCTKSWFWTRLAKALSLP
**SEQ** ID NO 2 (nucleotide sequence of binding region) 
5’CCCTTGGCTT GCAGGTGCCC ATCAGAAGCG ACTGATCCCC ATCAAGTACA
AGGCAATGAA GAAAG3’
SEQ ID NO 3 (crRNA Cr068), negative strand
   5'GCCTTGTACT TGATGGGGAT 3'
SEQ ID NO 4 (crRNA Cr069), negative strand
   5'GGGGATCGGTCGCTTCTGAT 3'
SEQ ID NO 5 (tracrRNA)
   5'GUUUUAGAGC UAUGCU3'
SEQ ID NO 6 (crRNA Cr068 + tracrRNA)
   5'GCCTTGTACT TGATGGGGAT GUUUUAGAGC UAUGCU3'
SEQ ID NO 7 (crRNA Cr069 + tracrRNA)
   5'GGGGATCGGTCGCTTCTGAT GUUUUAGAGC UAUGCU3'
SEQ ID NO 8 (crRNA Cr066 + tracrRNA)
   5'GTACTTGGAG ATCCGGCAAC GUUUUAGAGC UAUGCU3'
SEQ ID NO 9 (crRNA Cr067 + tracrRNA)
   5'ATGAAGGCAT CGAAACGCTC GUUUUAGAGC UAUGCU3'
SEQ ID NO 10 (primer P1 for Cr068 and Cr069-Cr149)
   5'TGGATTGTCAGCCTTCCCTC3'
SEQ ID NO 11 (primer P2 for Cr068 and Cr069-Cr149)
   5'GCCTTGTACTTGATGGGGAT3'
SEQ ID NO 12 (primer Cr066 P1)
   CTCAAGGGTAGAGGTGGGCA
SEQ ID NO 13 (primer Cr066 P2)
   GGGGACGTCCTCACCAATG
SEQ ID NO 14 (primer Cr067 P1)
   TCATCTTGGGAAGGGTGCAG
SEQ ID NO 15 (primer Cr067 P2)
   GCAGGTCCTGTGGCAACTTA

### BRIEF DESCRIPTION OF THE FIGURES

Fig. 1 shows that OCI-LY3 and BJAB lymphoma cells were electroporated with Cr066 (non-variant-specific), Cr067 (non-variant-specific) and Cr068 (variant L265P-specific).
Fig. 2 shows the editing efficiencies that were calculated by comparison of the Sanger sequencing chromatogram of the test sample with control cells treated with non-targeting crRNA (NTC) via Interference of CRISPR Edits (ICE) analysis.
Fig. 3 shows the cell viability that was calculated by normalizing the viability of test samples to the viability of corresponding non-targeting control (NTC) as percentage (CellTiter^{®}-Glo Cell Viability Assay, Promega).

### EXAMPLES

### Methods

### Cell culture conditions

Diffuse large B-cell lymphoma (DLBCL) OCI-LY3 (ACC 761) and BJAB (ACC 757) cells were purchased from DSMZ. These cells have been previously used often as *in vitro* models for the discovery of novel cancer therapeutic agents. Cells were cultured according to manufacturer's protocol in RPMI 1640 Medium (ATCC modification) (A1049101; Thermo Fisher Scientific) supplemented with heat-inactivated 20% fetal bovine serum (FBS) (A3160502; Thermo Fisher Scientific), and 1% penicillin-streptomycin solution (10378016; Thermo Fisher Scientific) (referred to as complete medium). Cells were cultured and maintained in an incubator at 37°C and 5% CO₂.

### Guide RNA design

gRNA targeting mutations were designed using SNP-CRISPT tool (https://www.flyrnai.org/tools/snp_crispr/). Using this tool, multiple SNPs can be uploaded for the design of gRNAs targeting single or multiple nearby mutations simultaneously. It designs all possible gRNAs for one variant based on the positioning of PAM. Moreover, Custom Alt-R^{®} CRISPR-Cas9 guide RNA tool (IDT) was used. On-target and off-target scores were evaluated using this tool.

### Nucleofections

The ribonucleoprotein (RNP) complex was made according to the manufacturer's instructions. Briefly, each Alt-R crRNA (IDT) and Alt-tracrRNA-ATTO550 (1072533, IDT) was reconstituted to 100 µM with Nuclease-Free Duplex Buffer (11-05-01-14, IDT). crRNA and tracrRNA oligos were mixed at equimolar concentrations in a sterile PCR tube to a final duplex concentration of 44 µM. Oligos were annealed by heating at 95°C for 5 min in PCR thermocycler and the mix was slowly cooled to room temperature. The crRNA-tracrRNA duplex (sgRNA) and Alt-R^{®} S.p. HiFi Cas9 Nuclease 3NLS (1078727, IDT) were precomplexed by combining and gently mixing by resuspending and incubated at room temperature for 10-20 min. sgRNAs were complexed with Cas9 at a molar ratio between 1:1 to 3:3 (sgRNA:Cas9) to form ribonucleoproteins (RNPs), which was always freshly prepared. To improve transfection efficiency, Alt-R electroporation enhancer (IDT) was added to the mix at a final concentration of 1.75 µM. About 5 x 10⁵ cells were resuspended in Neon electroporation buffer R and electroporated using the 10 µl Neon transfection system kit, with two pulses at 1400 V, width 20 ms. Transfected cells were incubated 48-72 h in pre-warmed RPMI-1640 medium supplemented with 20% FBS (A3160502; Thermo Fisher) (complete medium without antibiotics).

### Determination of editing efficiency and gene deletion/recombination

Cells were lysed and genomic DNA was extracted using QuickExtract^{™} DNA Extraction Solution (Lucigen; Biozym QE09050). For each target, PCR primers were designed to amplify the region flanking the targeted gDNA, generating a PCR amplicon of <900 bp. To screen for the presence of indels/mutations in the genomic region of interest, PCR was performed using GoTaq^{®} G2 Hot Start Taq Polymerase (M5122; Promega). PCR amplicon was purified using PureLink^{®} PCR Purification Kit (Invitrogen; K3100-02) or NucleoSpin Gel and PCR Clean-up kit (740609.250; Macherey-Nagel).

Sanger sequencing was then performed through a commercial vendor (Eurofins; Heidelberg) with one of the two primers used for amplification. The sequence traces obtained from Sanger sequencing were analysed by Interference of CIRPSR Edits (ICE), a tool that calculates overall editing efficiency and determines the profiles of all different types of CRISPR edits. By utilizing the indel patterns and their relative ratios provided by ICE default parameters, the control (NTC) trace sequence and a gene specific sgRNA edited trace sequence were aligned, allowing for the visualization of the indel patterns of each polyclonal population.

PCR was used to analyse the experiments where two different RNPs targeting two unique regions of the same or different gene thereby leading to a deletion and cancer specific chromosomal rearrangement. A confirmation of the presence of deletions could be obtained also by designing primers spanning the region to be deleted. In dual gRNA edited cells, the PCR band is only amplified upon successful editing by both crRNA. Without dual editing and subsequent rearrangement, the primers would be incapable of generating any PCR product.

### Cell viability assay

Immediately post nucleofection, cells were plated in a 96-well plate in a total of 100 µL medium containing RealTime-Glo MT cell viability reagents (G9711;RealTime-Glo MT Cell Viability Assay; Promega). Cells were incubated in a 37°C, 5% CO₂-humidified incubator (Thermo Fisher Scientific). After one hour incubation, the plate was read on a SpectraMax^{®} iD3 multi-mode microplate reader (Molecular Devices) set at 37°C to determine luminescence. Reading was taken again from the same plate at 72 h post nucleofection. The luminescence at 72h was normalized to 1h to compensate for any variations derived from pipetting.

### Statistical analysis

Statistical analysis was performed using Graphpad Prism software (GraphPad Software Inc., San Diego, CA, USA). For multiple group comparisons, ANOVA followed by Turkeys, Dunnett's or Sidak's multiple comparison test was performed depending on the data to be compared. Statistical tests and number of repetitions are described in the legends. Bar plots show mean ± standard error mean (SEM).

### Example 1: Targeting L265P with three crRNAs

### Purpose

Induction of cancer cell death in the lymphoma cell line OCI-LY3 via specific targeting of the hotspot mutation L265P by means of the CRISPR/Cas technology.

### Experiment

DLBCL cell lines OCI-LY3 (target cells) and BJAB (control cells) were nucleofected with ribonucleoprotein (RNP) complexes of the Cas9 protein and a guide RNA targeting *MYD88* (CRISPR/Cas complex; including one of three crRNAs: Cr066, Cr067 or Cr068). After 72 h treatment, cell viability assays were carried out using CellTiter^{®}-Glo Cell Viability Assay (Promega). Subsequently, cell samples were harvested, genomic DNA was isolated and investigated for the presence of site-specific gene modification by PCR followed by Sanger sequencing. Editing efficiency was determined via Interference of CRISPR Edits (ICE) analysis (Synthego).

### Results

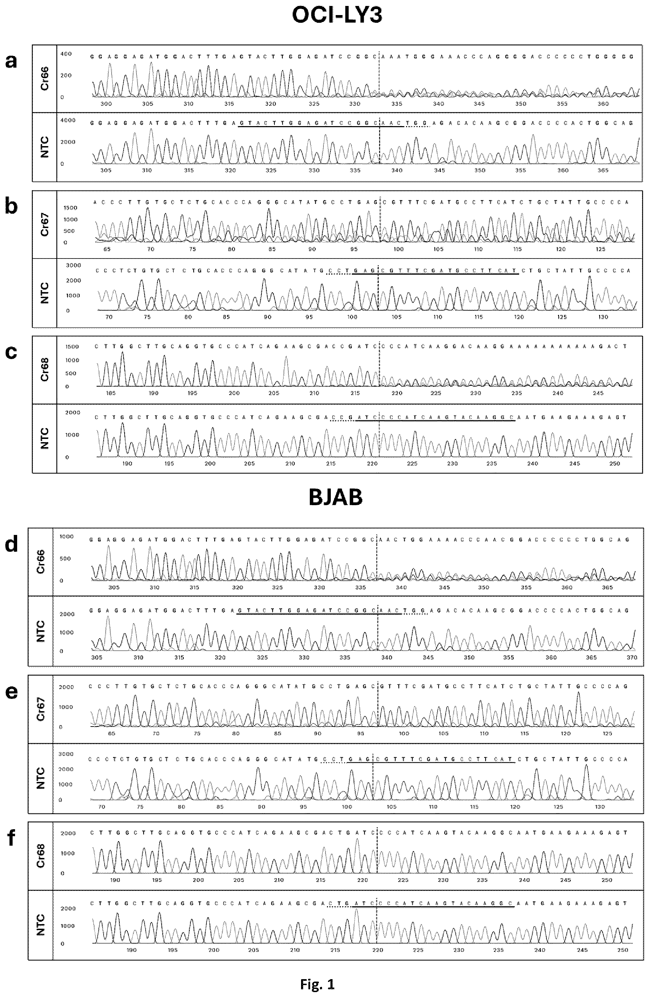

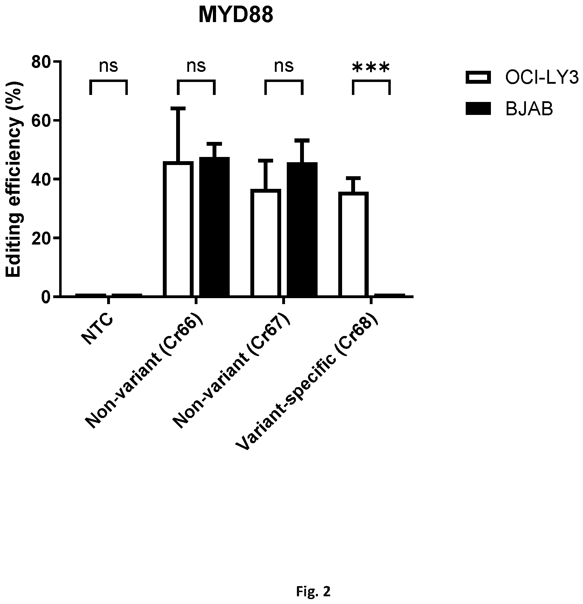

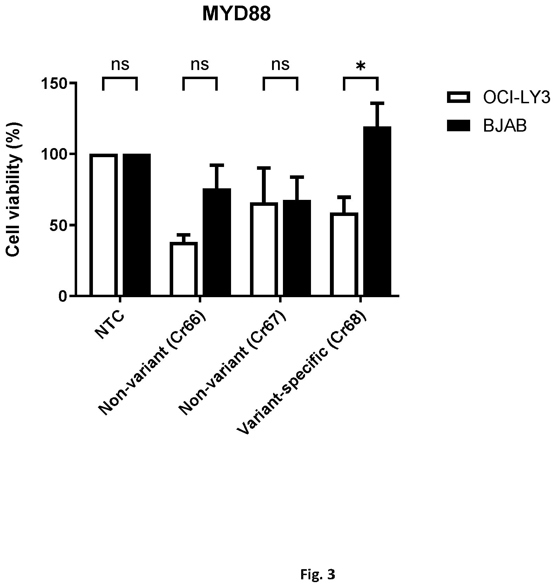

CRISPR/Cas complexes including one of three crRNAs (Cr066, Cr067 and Cr068) induced editing of *MYD88* in OCI-LY3 lymphoma cells (Fig. 1a-c), while only Cr066 and Cr067, targeting non mutated gene regions, mediated editing in BJAB cells (Fig. 1d-f). Cr068 targeting the mutation L265P caused 36% gene editing in OCI-LY3, while no statistically significant editing was observed in BJAB cells (Fig. 2). Moreover, cell viability assays revealed that crRNAs targeting wild-type *MYD88,* namely Cr066 and Cr067, reduced the cell viability considerably in both lymphoma cell lines OCI-LY3 and BJAB cells (76% up to 38% cell viability compared to corresponding non-targeting controls, NTC). Yet, treatment of cancer cells with Cr068, targeting L265P, restricted cell viability only in target OCI-LY3 cells (59% cell viability compared to NTC), while BJAB cells remained spared **(****Fig. 3****).** **Fig. 1** to **3** show that the targeting of hotspot L265P in gene *MYD88* causes mutations-specific gene editing.
**Fig. 1****:** OCI-LY3 (a-c) and BJAB **(d-f)** lymphoma cells (2,5 x 10⁵ cells) were electroporated with Cr066 (non-variant-specific), Cr067 (non-variant-specific) or Cr068 (variant L265P-specific). 72 h after electroporation, cells were collected and genomic DNA was isolated. The target sequence regions were amplified with primers flanking the expected edited points followed by Sanger sequencing of the amplicon. The traces show the edited and control (non-edited) Sanger traces in the area around the guide RNA binding sites. The horizontal black underlined region represents the guide sequence, and the horizontal dotted black region represents the PAM sequence. The cut site is depicted by a vertical black dotted line.
**Fig. 2****:** The editing efficiencies were calculated by comparison of the Sanger sequencing chromatogram of the test sample with control cells treated with non-targeting crRNA (NTC) via Interference of CRISPR Edits (ICE) analysis. Results are shown as mean ± SEM; *** p < 0.001.
**Fig. 3****:** Cell viability was calculated by normalizing the viability of test samples to the viability of corresponding non-targeting control (NTC) as percentage (CellTiter^{®}-Glo Cell Viability Assay, Promega). Results are shown as mean ± SEM; * p < 0.05.

### Discussion:

These results demonstrate that Cr068 can be used to specifically target and kill cancerous cells bearing the hotspot mutation L265P. By contrast, healthy or unrelated cells devoid of the mutation are not susceptible to gene editing and cell death. These findings substantiate the potential of CRISPR/Cas as a personalized therapy for cancer without inducing adverse effects derived from the unwanted killing of healthy cells.

### REFERENCES

1. Sung H, Ferlay J, Siegel RL, Laversanne M, Soerjomataram I, Jemal A, et al. Global Cancer Statistics 2020: GLOBOCAN Estimates of Incidence and Mortality Worldwide for 36 Cancers in 185 Countries. CA Cancer J Clin. 2021;71(3):209-49.
2. Hanahan D, Weinberg RA. Hallmarks of cancer: the next generation. Cell [Internet]. 2011 Mar;144(5):646-74. Available from: https://pubmed.ncbi.nlm.nih.gov/21376230/
3. Pitot HC. The Biology of Cancer. Second Edition. By Robert A. Weinberg. New York:
   Garland Science (Taylor & Francis Group). $150.00 (paper). xx + 876 p.; ill.; A:1-A:6;
   G:1-G:30; I:1-I:28 (index). ISBN: 978-0-8153-4220-5. [A DVD-ROM accompanies the book.] 2014.. Q Rev Biol. 2014;89(2).
4. Pasqualucci L, Dalla-Favera R. Genetics of diffuse large b-cell lymphoma. Blood. 2018; 131 (21).
5. Shankland KR, Armitage JO, Hancock BW. Non-Hodgkin lymphoma. Vol. 380, The Lancet. 2012.
6. Halwani AS, Panizo C, Isufi I, Herrera AF, Okada CY, Cull EH, et al. Phase 1/2 study of intratumoral G100 (TLR4 agonist) with or without pembrolizumab in follicular lymphoma. Leuk Lymphoma. 2022;63(4).
7. Fricker LD. Proteasome Inhibitor Drugs. Vol. 60, Annual Review of Pharmacology and Toxicology. 2020.
8. Sun Y, Zhao X, Ding N, Gao H, Wu Y, Yang Y, et al. PROTAC-induced BTK degradation as a novel therapy for mutated BTK C481S induced ibrutinib-resistant B-cell malignancies. Vol. 28, Cell Research. 2018.
9. Sahin U KSDMDJKMBCCJLMRBOT& de GJH. Personalized vaccines for cancer. 2012.
10. Sheikh IN, Ragoonanan D, Franklin A, Srinivasan C, Zhao B, Petropoulos D, et al. Cardiac relapse of acute lymphoblastic leukemia following hematopoietic stem cell transplantation: A case report and review of literature. Vol. 13, Cancers. 2021.
11. Wu HY, Cao CY. The application of CRISPR-Cas9 genome editing tool in cancer immunotherapy. Brief Funct Genomics. 2019;18(2).
12. Xing H, Meng L hua. CRISPR-cas9: a powerful tool towards precision medicine in cancer treatment. Vol. 41, Acta Pharmacologica Sinica. 2020.
13. Ahmad G, Amiji M. Use of CRISPR/Cas9 gene-editing tools for developing models in drug discovery. Vol. 23, Drug Discovery Today. 2018.
14. Gallipoli P, Giotopoulos G, Tzelepis K, Costa ASH, Vohra S, Medina-Perez P, et al. Glutaminolysis is a metabolic dependency in FLT3ITD acute myeloid leukemia unmasked by FLT3 tyrosine kinase inhibition. Blood. 2018;131(15).
15. Chen Y, Wen R, Yang Z, Chen Z. Genome editing using CRISPR/Cas9 to treat hereditary hematological disorders. Vol. 29, Gene Therapy. 2022.
16. Shuber A. P. Treating cancer with Cas endonuclease complexes . 2018.
17. Kawai T, Sato S, Ishii KJ, Coban C, Hemmi H, Yamamoto M, et al. Interferon-α induction through Toll-like receptors involves a direct interaction of IRF7 with MyD88 and TRAF6. Nat Immunol. 2004;5(10).
18. Semaan N, Alsaleh G, Gottenberg JE, Wachsmann D, Sibilia J. Etk/BMX, a Btk Family Tyrosine Kinase, and Mal Contribute to the Cross-Talk between MyD88 and FAK Pathways. The Journal of Immunology. 2008;180(5).
19. Campbell GR, To RK, Hanna J, Spector SA. SARS-CoV-2, SARS-CoV-1, and HIV-1 derived ssRNA sequences activate the NLRP3 inflammasome in human macrophages through a non-classical pathway. iScience. 2021;24(4).
20. Yamamoto T, Tsutsumi N, Tochio H, Ohnishi H, Kubota K, Kato Z, et al. Functional assessment of the mutational effects of human IRAK4 and MyD88 genes. Mol Immunol. 2014;58(1).
21. Ohnishi H, Tochio H, Kato Z, Orii KE, Li A, Kimura T, et al. Structural basis for the multiple interactions of the MyD88 TIR domain in TLR4 signaling. Proc Natl Acad Sci USA. 2009;106(25).
22. Bonnert TP, Garka KE, Parnet P, Sonoda G, Testa JR, Sims JE. The cloning and characterization of human MyD88: A member of an IL-1 receptor related family. FEBS Lett. 1997;402(1).
23. Larkin MA, Blackshields G, Brown NP, Chenna R, Mcgettigan PA, McWilliam H, et al. Clustal Wand Clustal X version 2.0. Bioinformatics. 2007;23(21).

## Claims

1. Method for the manufacture of individualized CRISPR/Cas complexes comprising the steps
a) identifying in a tumor specimen of a human cancer patient a mutation at position 38.141.150 on chromosome 3,
b) preparing for the mutation identified in a) an individualized CRISPR/Cas complex, wherein the individualized CRISPR/Cas complex comprises a guide RNA and a Cas endonuclease, wherein the CRISPR/Cas complex targets the mutation.

2. Method according to claim 1, wherein the guide RNA for the individualized CRISPR/Cas complex is equal to or is complementary to a polynucleotide with the nucleotide sequence as shown in SEQ ID NO 2 or a part thereof.

3. Method according to claim 2, wherein the mutation at position 38.141.150 is enclosed in the nucleotide sequence or within the PAM of the respective Cas enzyme.

4. Method according to any of the preceding claims, wherein the guide RNA comprises from 64 to 16 nucleotides, preferably from 40 to 18 nucleotides, more preferably from 36 to 20 nucleotides, even more preferably from 24 to 20 nucleotides.

5. Method according to any of the preceding claims,
wherein the guide RNA comprises
i) a crRNA and
ii) a tracrRNA, and
wherein preferably the crRNA comprises or consists of a sequence according to SEQ ID NO 3 or a variant therof or a sequence according to SEQ ID NO 4 or a variant therof.

6. Method according to any of the preceding claims, wherein the tumor specimen is from B-cell-lymphocytes.

7. Method according to any of the preceding claims, wherein the individualized CRISPR/Cas complex comprises the guide RNA and the Cas endonuclease in a ratio of 3:1 to 1:1.

8. Method according to any of the preceding claims, wherein the Cas endonuclease is a type I, type II or type III endonuclease, preferably a type I endonuclease, most preferably a CRISPR/Cas9 endonuclease.

9. Method, preferably *in-vitro* method, for inducing cell death or impairment of cell proliferation in cancerous or pre-cancerous cells of B-cell-lymphocytes, comprising the steps:
a) isolation of B-cell-lymphocytes from one or more specimen obtained from a human cancer patient or a human subject supposed to have or to develop cancer, whereby non-cancerous B-cell-lymphocytes and cancerous or pre-cancerous B-cell-lymphocytes are obtained;
b) cultivation, preferably *in-vitro* cultivation, of the B-cell-lymphocytes from the one or more specimen obtained in a);
c) identifying in the cancerous or pre-cancerous B-cell-lymphocytes a cancer specific mutation at position 38.141.150 on chromosome 3,
d) preparing an individualized CRISPR/Cas complex for the cancer specific mutation identified in c) according to step b) of the method for the manufacture of individualized CRISPR/Cas complexes according to at least one of claims 1 to 8,
e) adding the prepared individualized CRISPR/Cas complex to the cultivated B-cell-lymphocytes obtained in step b), and
f) optionally, determining if cell death or impairment of cell proliferation is induced in the cancerous or pre-cancerous B-cell-lymphocytes of said cultivated B-cell-lymphocytes and/or preferably determining if cell death or impairment of cell proliferation is not induced in the non-cancerous B-cell-lymphocytes of said cultivated B-cell-lymphocytes.

10. Individualized CRISPR/Cas complex comprising a guide RNA and a Cas endonuclease that is equal to or is complementary to a polynucleotide with the nucleotide sequence as shown in SEQ ID NO 2 or a part thereof.

11. Individualized CRISPR/Cas complex according to claim 10,
wherein the guide RNA comprises or consists of
i) a crRNA according to SEQ ID NO 3 or a variant therof or a crRNA according to SEQ ID NO 4 or a variant therof and
ii) a tracrRNA.

12. Composition, preferably pharmaceutical composition, comprising one or more individualized CRISPR/Cas complexes manufactured according to the method of any of claims 1 to 8 or according to any of claims 10 to 11.

13. Individualized CRISPR/Cas complex manufactured according to the method of any of claims 1 to 8, individualized CRISPR/Cas complex according to claim 10 or 11 or composition according to claim 12, for use in the treatment of B-cell-lymphoma, preferably for use in the treatment of diffuse large B-cell-lymphoma, more preferably for use in the treatment of Germinal center B-cell-like diffuse large B-cell-lymphoma or Activated B-cell-like diffuse large B-cell-lymphoma.

14. Individualized CRISPR/Cas complex manufactured according to the method of any of claims 1 to 8, individualized CRISPR/Cas complex according to claim 10 or 11 or composition according to claim 12, for inducing cell death or impairment of cell proliferation in cancerous or pre-cancerous B-cell-lymphocytes.

15. Individualized CRISPR/Cas complex for use in the treatment of cancer, preferably for use in inducing cell death or impairment of cell proliferation in cancerous or pre-cancerous cells of B-cell-lymphocytes, wherein the individualized CRISPR/Cas complex is manufactured according to a method, preferably according to an *in-vitro* method, comprising the steps:
a) isolation of B-cell-lymphocytes from one or more specimen obtained from a human cancer patient or a human subject supposed to have or to develop cancer, whereby non-cancerous B-cell-lymphocytes and cancerous or pre-cancerous B-cell-lymphocytes are obtained;
b) cultivation, preferably *in-vitro* cultivation, of the B-cell-lymphocytes from the one or more specimen obtained in a);
c) identifying in the cancerous or pre-cancerous B-cell-lymphocytes a cancer specific mutation at position 38.141.150 on chromosome 3,
d) preparing an individualized CRISPR/Cas complex for the cancer specific mutation identified in c) according to step b) of at least one of claims 1 to 8.
